Europäisches Patentamt

European Patent Office

Office européen des brevets

(19)

(11) **EP 0 562 107 B1**

(12) **FASCICULE DE BREVET EUROPEEN**

(45) Date de publication et mention
de la délivrance du brevet:
**02.05.2002 Bulletin 2002/18**

(21) Numéro de dépôt: **92923831.9**

(22) Date de dépôt: **09.10.1992**

(51) Int Cl.[7]: **C08B 37/00**, A61K 39/02

(86) Numéro de dépôt international:
**PCT/FR92/00955**

(87) Numéro de publication internationale:
**WO 93/07178 (15.04.1993 Gazette 1993/10)**

(54) **OLIGOSIDE DERIVE D'UN POLYOSIDE ANTIGENIQUE ISSU D'UN AGENT PATHOGENE**

OLIGOSIDDERIVAT AUS EINEM POLYOSIDANTIGEN VON EINEM PATHOGENEN ERREGER

OLIGOSIDE DERIVED FROM AN ANTIGEN POLYOSIDE OBTAINED FROM A PATHOGENIC
AGENT

(84) Etats contractants désignés:
**AT BE CH DE DK ES FR GB GR IE IT LI LU MC NL
SE**

(30) Priorité: **10.10.1991 FR 9112478**

(43) Date de publication de la demande:
**29.09.1993 Bulletin 1993/39**

(73) Titulaire: **Aventis Pasteur
69367 Lyon Cedex 07 (FR)**

(72) Inventeur: **MOREAU, Monique
F-69007 Lyon (FR)**

(74) Mandataire: **Tonnellier, Jean-Claude
NONY & ASSOCIES
3, rue de Penthièvre
75008 Paris (FR)**

(56) Documents cités:
**EP-A- 0 097 407          EP-A- 0 245 045
BE-A- 1 000 118**

- **AGRICULTURAL AND BIOLOGICAL
  CHEMISTRY. vol. 50, no. 10, Octobre 1986,
  TOKYO JP pages 2579 - 2583 K. UCHIDA ET S.
  KAWAKISHI 'Oxidative depolymerisation of
  polysaccharides induced by the ascorbic
  acid-copper ion systems'**
- **JOURNAL OF BIOLOGICAL CHEMISTRY. vol.
  265, no. 30, Octobre 1990, BALTIMORE US pages
  18278 - 18283 L.C. PAOLETTI ET AL. 'An
  oligosaccharide-tetanus toxoid conjugate
  vaccine against type iii group b streptococcus'**
- **CARBOHYDRATE RESEARCH. vol. 152, 1986,
  AMSTERDAM NL pages 7 - 20 S.C. SZU ET AL.
  'Ultrasonic irradiation of bacterial
  polysaccharides, characterization of the
  depolymerized products and some applications
  of the process'**

**Description**

La présente invention a pour objet un oligoside dérivé d'un polyoside antigénique issu d'un agent pathogène, son procédé de préparation et son usage notamment à titre d'agent vaccinal.

Les bactéries ainsi que des champignons tels que des levures incorporent des polyosides dans leur structure de surface. Ainsi la grande majorité des bactéries sont recouvertes d'un exsudat de nature polyosidique qui est fixé à la bactérie de manière plus ou moins forte mais qui n'est pas à proprement parler une enveloppe. Cet exsudat porte le nom de capsule ou de glycocalyx. D'autre part la membrane externe des bactéries gram-négatives est entre autre constituée de lipopolysaccharide (LPS). Enfin, des polysaccharides se retrouvent aussi dans la paroi des champignons. Ces polyosides sont en fait des antigènes de surface qui induisent une réponse immunologique chez un mammifère infecté.

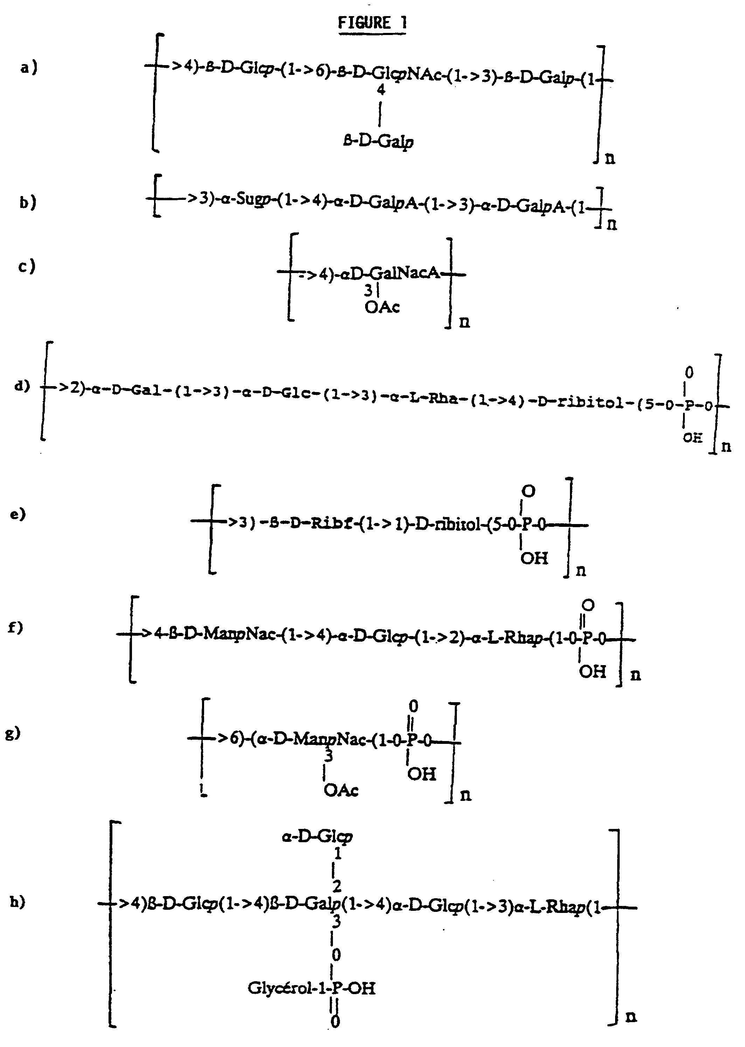

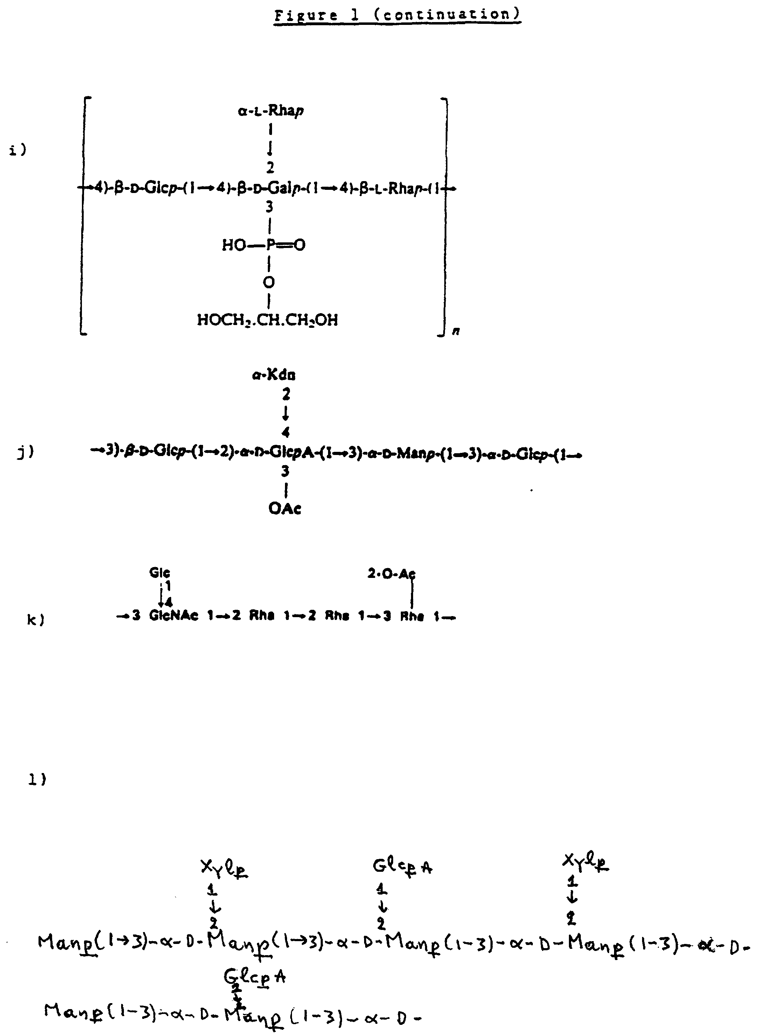

De tels polyosides sont constitués sur la base d'unités répétitives, dans lesquelles les constituants et les liaisons sont définis et qui sont chacune caractéristiques de l'espèce fongique ou bactérienne considérée. Ces unités répétitives contiennent les épitopes, les structures déterminantes de l'antigénicité. A titre d'illustration, on a reporté dans la Figure 1, différents types d'unités répétitives provenant de polyosides capsulaires ou de lipopolysaccharides. Les unités répétitives contiennent souvent des fonctions très labiles telles que par exemple des fonctions phosphates, présentes dans le squelette de la molécule ou en position ramifiée ainsi que, par exemple des groupes O-acétyles et pyruvates.

Un polyoside est en fait constitué d'un ensemble de molécules polymères contenant des unités osidiques en nombre pouvant varier d'une molécule à une autre. Par conséquent, un polyoside ne peut être décrit en terme de poids moléculaire que par un poids moléculaire moyen. Dans le cas d'un homopolyoside, les unités osidiques sont des monosaccharides. Dans le cas d'un hétéropolyoside les unités osidiques forment un enchaînement de constituants liés entre eux de manière définie.

En ce qui concerne le poids moléculaire moyen d'un polyoside, il peut être apprécié par filtration sur gel. Le poids moléculaire est alors exprimé en terme de constante d'élution ($K_D$) ou d'équivalent dextran (EqD) selon la méthode décrite par Granath et al, J. Chrom. (1967) 28 : 69 que l'on résume comme suit :

Un polyoside en solution est analysé sur une colonne de gel de chromatographie d'exclusion-diffusion qui sépare des éléments en fonction de leur poids moléculaire. Ce type de gel est disponible dans le commerce, par exemple sous les noms commerciaux de Séphadex (Pharmacia), Bio-Gel (Bio-Rad), Fractogel (Toyo-Soda), Sépharose (Pharmacia), et Séphacryl (Pharmacia). La zone de fractionnement du gel doit être bien évidemment adaptée à la gamme des poids moléculaires représentés au sein de la population moléculaire à analyser. A titre d'exemple, on indique que les colonnes de gel de filtration Sepharose 2BCL, 4BCL et 6BCL ont une zone respective de fractionnement déterminée en équivalent dextran de 20.000.000 à 100.000 ; 5.000.000 à 20.000 et 1.000.000 à 10.000. De manière générale, on utilise comme éluant une solution saline, par exemple du chlorure de sodium 0,2 M. Une constante d'élution se calcule selon la formule suivante :

$$\frac{Ve - Vo}{Vt - Vo}$$

dans laquelle

Ve est le volume d'élution de la préparation considérée ; Vt est le volume total de la colonne ; et Vo est le volume mort de la colonne.

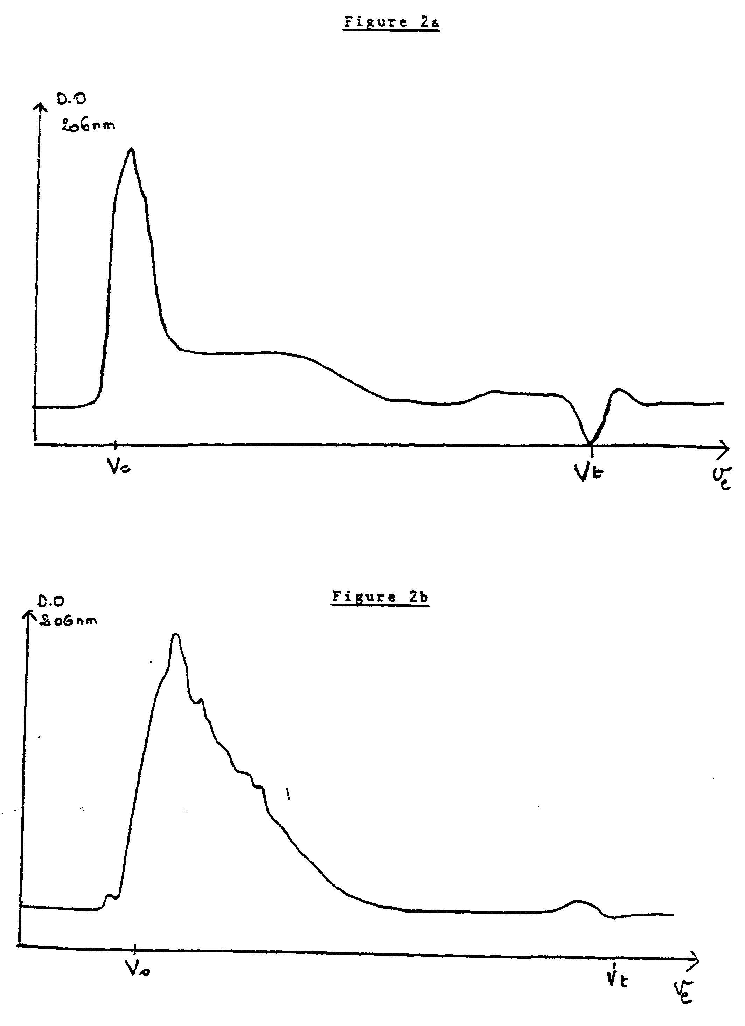

A titre d'exemple les Figures 2a et 2b présentent les profils d'élution respectifs des polyosides de *Salmonella typhi* et de *Streptococcus pneumoniae* type 1 sur une colonne de Sépharose 4BCL. Le polyoside de *S. typhi* est donc composé de polymères dont les poids moléculaires les plus élevés ne sont pas appréciables sur Sépharose 4BCL. De même, le polyoside de *S. pneumoniae* type 1 est essentiellement composé de polymères dont les poids moléculaires varient d'un $K_n$ nul à 0,45. Par définition, la constante d'élution moyenne du polyoside s'apprécie au point d'intersection des tangentes des pentes du profil d'élution ; soit de manière approximative, au sommet du profil d'élution. Ainsi, la constante d'élution moyenne des polyosides présentés aux Figures 2a et 2b est respectivement zéro et 0,04.

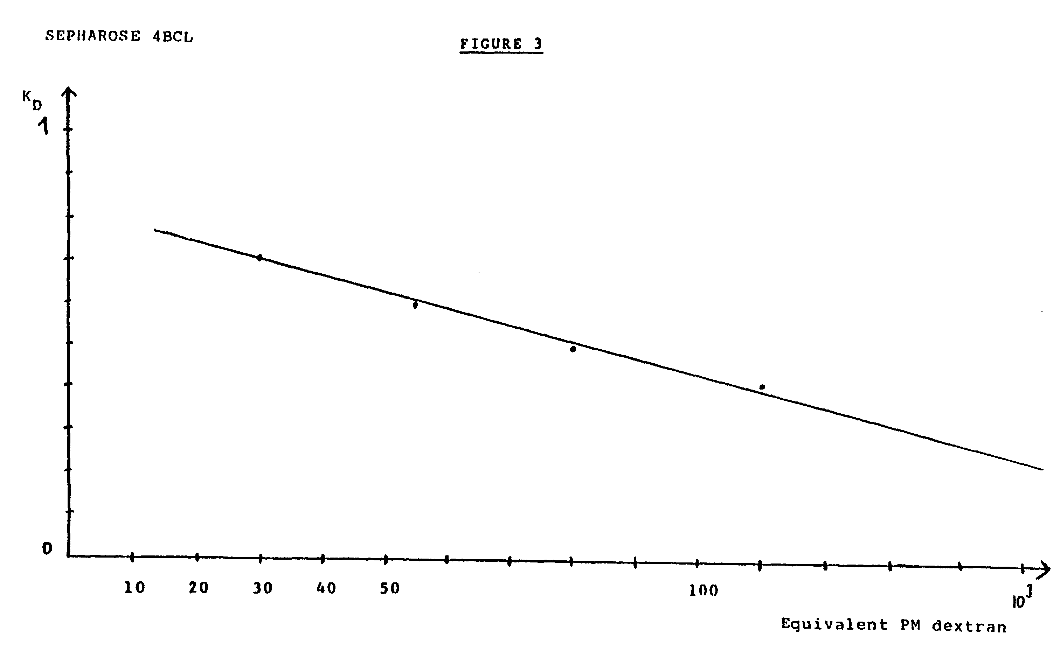

Sur la base d'une gamme-étalon établie en utilisant du dextran, on peut convertir une constante d'élution en poids moléculaire exprimé en EqD. A titre d'exemple, la Figure 3 présente un modèle de gamme-étalon.

En raison de leur pouvoir antigénique, les polyosides, antigènes de surface d'agents pathogènes e.g. bactériens ou fongiques sont de bons candidats à titre d'agent vaccinal. Des vaccins comprenant un extrait polyosidique se sont en effet révélés efficaces chez les adultes. Toutefois tel n'est pas le cas chez les jeunes enfants. Afin de surmonter ce désagrément majeur, on a proposé avec succès de lier les polyosides de manière covalente à des protéines, ce qui confère aux molécules ainsi obtenues un caractère T-dépendant et augmente leur immunogénicité.

Parmi les vaccins à base de polyosides on compte à ce jour des vaccins contre les infections à *Neisseria meningitidis* groupe A, C, Y ou W 135 à *Streptococcus pneumoniae*, à *Haemophilus influenzae* type B et à *Salmonella*

*typhi.*

Bien que les polyosides antigéniques d'agents pathogènes soient potentiellement intéressants à titre d'immunogènes, leur emploi se révèle délicat en raison de l'importance de leur poids moléculaire moyen qui peut atteindre plusieurs millions d'équivalent-dextran. En particulier, lorsque l'on souhaite utiliser un polyoside sous forme de conjugué, c'est-à-dire couplé à une protéine, on se heurte à des problèmes techniques difficiles à résoudre. Au cours du processus de couplage, un gel ou un floculat peut se former, rendant ainsi le conjugué difficile à stériliser par filtration.

Afin de surmonter cet écueil, on a déjà proposé de réduire la taille des polyosides pour les rendre plus aisément manipulables. A cette fin, on a préconisé diverses méthodes de clivage. Ainsi il existe par exemple des méthodes bien établies telles que une fragmentation aux ultra-sons ou par hydrolyse en milieu acide, basique ou enzymatique. Toutefois, ces méthodes de fragmentation sont loin d'être satisfaisantes. En effet, elles conduisent trop souvent à la destruction totale ou partielle des épitopes caractéristiques par perte de fonctions chimiques essentielles pour la spécificité antigénique.

A titre d'illustration de l'état de la technique discuté ci-dessus, on peut citer les documents PAOLETTI et al., J. Biol. Chem. (1990) 265 (30), 18278-18283, EP-A-97407 et EP-A-245045, qui décrivent des procédés de dépolymérisation de polyosides d'agents pathogènes, la dépolymérisation étant effectuée par hydrolyse acide, par hydrolyse enzymatique ou encore par oxydation periodique.

L'hydrolyse acide ou basique du polyoside capsulaire de *S. typhi* ou de *N. meningitidis* groupe A élimine les groupements acétyles qui sont nécessaires à la spécificité antigénique du polyoside. De plus la réacétylation correcte des fragments obtenus par hydrolyse est très difficile sinon impossible.

D'autre part, avec la méthode de fragmentation par hydrolyse, il n'est pas toujours possible d'obtenir des fragments de taille relativement homogène, alors que la pratique pharmaceutique requiert des produits homogènes, de qualité constante et standardisée.

En ce qui concerne la fragmentation aux ultra-sons, on a par exemple montré que les groupements phosphates terminaux du polyoside d'*H. influenzae* type B sont perdus par traitement aux ultra-sons. Ceci peut laisser craindre que des groupements phosphates en position ramifiée sur les chaînes polyosidiques soient de même éliminés au cours d'un tel traitement.

En plus, bien que des fragments de taille relativement homogène puissent être obtenus aux ultra-sons, l'obtention de fragments de taille suffisamment faible par la méthode aux ultra-sons nécessite un temps de traitement très long, ce qui peut provoquer entre autres inconvénients, une détérioration rapide de l'appareillage. Son efficacité étant limitée, l'application des ultra-sons à l'échelle industrielle est à exclure.

Finalement, l'utilisation de la dépolymérisation enzymatique des polyosides se limite aux polyosides pour lesquels des enzymes appropriées sont connues. Cet inconvénient majeur est toutefois inhérent au caractère hautement spécifique des enzymes vis-à-vis de leur substrat.

Pour le domaine vaccinal un progrès technique et économique important serait donc de pouvoir disposer d'un procédé de dépolymérisation qui ne présente plus les divers inconvénients des procédés de l'état de la technique. En d'autres termes, il faudrait notamment pouvoir disposer d'un procédé de dépolymérisation de polyosides antigéniques qui présente un ensemble de propriétés avantageuses parce que :

- Il permet d'obtenir des oligosides ayant un nombre d'unités osidiques réduit tout en conservant les déterminants structurels essentiels d'au moins un épitope du polyoside à fragmenter.

- Il est possible de l'utiliser pour n'importe quelle structure polyosidique antigénique envisagée.

- Il permet d'obtenir des fragments de taille suffisament homogène, et

- Il est d'un faible coût et d'une très grande simplicité de mise en oeuvre.

De manière surprenante, on a maintenant trouvé que ces objectifs sont atteints par une réaction de dépolymérisation par oxydo-réduction.

Jusqu'à présent, une telle méthode, souvent désignée en langue anglaise sous le sigle ORD, a été mise en oeuvre pour fragmenter des polyosides tels que l'héparine, l'acide hyaluronique, le dextran et l'ADN et cela à des fins totalement différentes de celles poursuivies par la présente demande. Il s'agissait par exemple de réduire la viscosité des produits ou, dans le cas d'une molécule linéaire telle que l'héparine d'obtenir des fragments de taille réduite dont il était connu qu'ils ont une activité anticoagulante différente. Bien entendu, comme les produits auxquels cette méthode était appliquée ne présentaien, pas d'intérêt immunogénique, l'étude de l'intégrité des unités répétitives des fragments obtenus n'était pas abordée. A titre d'illustration de cet état de la technique, on peut citer les documents USHIDA et KAWAKISHI, Agric. Biol. Chem. (1986) 50 (10), 2579-2583 et BE-A-1000118.

D'autre part, il existe de nombreuses publications antérieures telles que par exemple, K. Uchida, Carbohydrate Research, (1988) 173 : 89-99 ; H. Uchiyama, Journal of Biological Chemistry, (1990) 265 : 7753-7759 ; S.W. Green, The Carbohydrates Chemistry and Biochemistry IB ; Academic Press (1980) : 1132 ; A. Herp, The Carbohydrates Chemistry and Biochemistry IB ; Academic Press (1980) : 1276 ; Kochetkov et al, Radiation Chemistry of Carbohydrates, Pergammon Prese, Oxford, (1979) qui décrivent la réaction ORD comme destructrice soit des groupes phosphates, soit des cycles carbohydrates par clivage des liaisons carbone-carbone du cycle.

L'impression d'ensemble qui s'est dégagée de l'état antérieur de la technique a fait que la réaction ORD est généralement reconnue comme destructive.

Rien ne laissait donc penser que l'ORD serait meilleure que par exemple la méthode par hydrolyse ou par traitement aux ultra-sons en vue des buts recherchés i.e., la conservation des épitopes.

C'est pourquoi, avant la présente invention il n'avait jamais été démontré qu'il était possible d'obtenir des oligosides, dont la structure de l'unité répétitive caractéristique est conservée, par fragmentation oxydo-réductive des polyosides respectifs, afin de pouvoir les utiliser à titre d'agent actif dans des formulations permettant de renforcer les défenses immunitaires d'un individu.

On a maintenant découvert qu'il est possible de mettre en oeuvre la méthode ORD pour obtenir des oligosides ayant conservé au moins un déterminant antigénique du polyoside de départ.

Par ailleurs, on a aussi constaté que la méthode de dépolymérisation par oxydo-réduction appliquée à un polyoside antigénique permettait d'obtenir dans de bonnes conditions des fragments tout à fait satisfaisants du point de vue de l'homogénéité de la taille, malgré le fait qu'il est connu que la réaction ORD est une réaction radicalaire clivant le polyoside de manière aléatoire.

En conséquence, l'invention propose un oligoside ayant conservé au moins un déterminant antigénique d'un polyoside antigénique issu d'un agent pathogène, caractérisé en ce qu'il est préparé par un procédé comprenant les étapes consistant à (i) soumettre ledit polyoside à une réaction de dépolymérisation par oxydo-réduction, (ii) récolter l'oligoside ainsi obtenu et, si désiré, (iii) le coupler à un partenaire de conjugaison ou à un porteur pour obtenir ledit oligoside sous la forme d'un conjugué ou lié à un porteur.

Par "oligoside", on entend un ensemble de molécules ; chaque molécule ayant un nombre réduit d'unités osidiques, par rapport au polyoside de départ, et contenant par exemple de 4 à 500 unités osidiques.

Le poids moléculaire moyen d'un oligoside est défini selon les règles explicitées ci-avant pour un polyoside. Généralement, un oligoside selon l'invention peut avoir une constante d'élution moyenne sur une colonne Sépharose 4BCL de 0,3 à 0,9, en particulier de 0,4 à 0,8, par exemple de 0,6 à 0,7. Autrement exprimé, un tel oligoside a un poids moléculaire moyen de 200.000 à 2.000 EqD, notamment de 150.000 à 10.000 EqD, en particulier de 60.000 à 30.000 EqD.

Selon un aspect préféré de la présente invention, le polyoside antigénique est un antigène de surface issu d'un agent pathogène qui peut être un champignon ainsi qu'une bactérie Gram-négative ou Gram-positive, notamment du genre *Staphylococcus, Streptococcus, Klebsiella, Salmonella, Escherichia, Neisseria, Shigella ou Haemophilus*.

L'agent pathogène bactérien *est par exemple Streptococcus pneumoniae, Neisseria meningitidis, Salmonella typhi ou Haemophilus influenzae.*

L'agent pathogène fongique peut être une levure, plus particulièrement sélectionnée parmi les genres *Candida, Cryptococcus*, Hansenula, Lipomyces, Rhinocladiella et Rhodotorula.

Aux fins de la présente invention, une réaction d'oxydo-réduction est mise en oeuvre en présence d'au moins un système oxydo-réducteur qui peut être notamment sélectionné parmi des thiols e.g., glutathion, cystéine ; l'oxygène ; l'hydroquinone ; des ions de métaux à plusieurs valences, e.g., fer et cuivre ; l'acide ascorbique, le peroxyde d'hydrogène ; ces deux derniers étant préférés.

Les différents paramètres expérimentaux qui peuvent influencer la cinétique de la réaction (concentration des produits, pH, température, temps de réaction) peuvent être facilement déterminés par l'homme du métier en fonction des caractéristiques du polyoside de départ, du système d'oxydoréduction sélectionné, et de la taille moyenne des fragments qu'il souhaite obtenir. Lorsque ces fragments sont destinés notamment à des fins vaccinales, l'homme du métier prendra en particulier soin d'ajuster les différents paramètres de manière à obtenir des fragments d'une taille moyenne convenable, c'est-à-dire conservant un bon pouvoir antigénique et éventuellement adaptés à la mise en oeuvre de conjugués ; par exemple, d'un ordre de grandeur tel que précédemment indiqué. D'une manière générale, les conditions réactionnelles optimales peuvent être déterminées lors de tests de routine. A titre d'indication, on précise toutefois dans les exemples ci-après des valeurs expérimentales permettant d'obtenir de bons résultats.

Un polyoside destiné à être soumis à un procédé selon l'invention peut être extrait et purifié selon des méthodes connues, en particulier par précipitation à l'alcool ou au bromure de cétyltriméthylammonium ou par filtration sur gel. Pour une revue de ces méthodes, on se refère en particuliel à R.I. Whistler, in Methods in Carbohydrate Chemistry, (1965) I : 3-62.

Le polyoside est avantageusement préparé en solution aqueuse à une concentration de 5 mg/ml. On indique qu'une concentration avantageuse peut varier de 0,5 à 50 mg/ml, en particulier de 0,5 à 10 mg/ml. Une telle solution aqueuse est utilisable comme produit de départ.

Un système oxydo-réducteur tel que défini ci-dessus est ajouté à la préparation polyosidique en une seule fois ou de façon continue ou discontinue dans un rapport pondéral final système oxydo-réducteur : polyoside pouvant varier notamment de 0,01 à 10, en particulier de 0,1 à 5, par exemple de 0,1 à 1.

Un procédé selon l'invention peut être mis en oeuvre à un pH de 3 à 8,5, en particulier de 3 à 6,5. De même, la température du milieu réactionnel n'est pas critique ; elle peut notamment varier de 4 à 60°C, en particulier de 18 à 40°C.

Le temps nécessaire pour parvenir à une fragmentation souhaitée du polyoside est généralement de 30 min à 1 h sauf cas particulier ; par exemple, pour le polyoside Vi de *S. typhi*, le temps de réaction est sensiblement plus long. Le temps de réaction peut être adapté en fonction de la taille des fragments que l'on souhaite obtenir.

Selon un mode de réalisation particulier, à une préparation aqueuse d'un polyoside ayant une concentration de 0,5 à 50 mg/ml, en particulier de 0,5 à 10 mg/ml, on ajoute de l'acide ascorbique jusqu'à une concentration finale de 0,01 à 25 mg/ml, en particulier de 0,1 à 10 mg/ml. L'oxygène naturellement dissous dans le milieu réactionnel est généralement suffisant ; si tel n'était pas le cas, on pourrait en injecter dans le milieu. Afin d'accélérer la réaction, on opère de préférence, en présence d'un sel soluble d'un métal à plusieurs degrés d'oxydation, tel que le fer ou le cuivre. Le ou les sel(s) métallique(s) peut(vent) être ajouté(s) à une concentration de 1 $\mu$M à 100 mM, en particulier de 1 à 10 mM.

Selon un mode de réalisation alternatif à celui énoncé ci-dessus, on remplace l'acide ascorbique par du peroxyde d'hydrogène qui peut être ajouté jusqu'à une concentration de 0,1 à 100 mM, en particulier de 0,5 à 10 mM, éventuellement en présence d'un sel métallique.

Un oligoside préparé en soumettant un polyoside à une réaction de dépolymérisation par ORD est constitué d'un ensemble de molécules dont la constante d'élution moyenne est inférieure à celle du polyoside dont il dérive par fragmentation (la comparaison étant bien évidemment mise en oeuvre en utilisant une même colonne de chromatographie). L'oligoside peut être isolé selon une technique conventionnelle, par exemple par précipitation à l'aide d'un agent précipitant approprié e.g., acétone ou alcool, par filtration sur membrane ayant un seuil de séparation approprié, par chromatographie d'exclusion-diffusion ou d'échange d'ions. Par la suite, on peut aussi choisir de n'utiliser que certaines fractions de l'oligoside contenant des molécules ayant une constante d'élution égale à, ou aux alentours de la constante d'élution moyenne.

De manière optionnelle, l'oligoside selon l'invention peut être couplé par liaison covalente à un composé de nature peptidique, protéique ou à un autre polymère organique comme par exemple le polyacrylate pour former un conjugué capable de favoriser l'immunogénicité de l'oligoside notamment chez un mammifère. Le partenaire de conjugaison peut être notamment une protéine bactérienne, par exemple une toxine, l'anatoxine correspondante ou une sous-unité d'une toxine multimérique ainsi qu'une protéine de membrane, une sous-unité d'une protéine de membrane multimérique ou une protéine cytoplasmique. A titre d'exemple, on cite la toxine *Pertussis*, la toxine cholérique, la toxine tétanique et la toxine diphtérique. Ces protéines peuvent être extraites à partir des bactéries d'origine ou bien être obtenues par voie recombinante.

Une réaction de couplage, par liaison covalente, d'un oligoside avec un partenaire de conjugaison de façon à obtenir un conjugué utilisable en tant que vaccin peut être mise en oeuvre de manière conventionnelle. On peut par exemple faire apparaître sur l'oligoside une fonction capable de réagir avec un groupement fonctionnel du partenaire de conjugaison. On peut également faire réagir un agent de couplage bi-fonctionnel avec l'oligoside puis avec un partenaire de conjugaison, ou inversement. Pour une revue de ces diverses méthodes de couplage, on se réfère en particulier à W.E. Dick et M. Beurret, in Conjuguâtes Vaccines, J.M. Cruse, R.E. Lewis Jr Eds, Contrib. Microbiol. Immunol. Basel, Karger, (1989) <u>10</u> : 48. Par ailleurs, le procédé de fragmentation par oxydo-réduction introduit des groupements réducteurs, notamment dans les oligosides dérivés des polyosides de *S. pneumoniae* type 6B et 19F, *H. influenzae* et *N. meningitidis* groupe A. Ceci permet donc d'utiliser la technique de conjugaison par amination réductrice.

L'immunogénicité d'un oligoside selon l'invention peut également être favorisée en utilisant des liposomes porteurs auxquels les oligosides sont liés de manière covalente. Favorisent également l'immunogénicité, des vecteurs, qui retiennent l'oligoside par incorporation à l'aide de forces ioniques ou hydrophobes, tels que par exemple les cyclodextrines, les liposomes et les iscoms.

L'invention concerne donc un oligoside tel que défini précédemment présenté sous diverses formes, en particulier sous la forme d'un conjugué, ou couplé à un liposome porteur, ou incorporé dans un vecteur. Selon un aspect préféré de la présente invention, l'oligoside est sous forme de conjugué.

Un oligoside selon l'invention est notamment utile pour renforcer les défenses immunitaires d'un individu, chez les mammifères et notamment chez les humains, par exemple pour prévenir ou atténuer les effets d'une infection induite par un agent pathogène e.g., une infection bactérienne ou une mycose. Sont également utiles, les mélanges d'oligosides selon l'invention dérivés de différents polyosides antigéniques, ainsi que les mélanges d'un oligoside selon l'invention avec des agents actifs autres qu'un oligoside selon l'invention. De tels mélanges se prêtent à la préparation de vaccins polyvalents.

En conséquence, l'invention propose également :

- Une composition pharmaceutique qui comprend à titre d'agent actif au moins un oligoside selon l'invention ; la composition est notamment un vaccin ;

- L'usage thérapeutique d'un oligoside selon l'invention ;

- Une méthode pour renforcer les défenses immunitaires d'un individu e.g., de vaccination, qui comprend l'acte d'administrer, en quantité suffisante d'un point de vue thérapeutique, au moins un oligoside selon l'invention, à un sujet ayant besoin d'un tel traitement e.g., d'une telle vaccination ;

- L'utilisation d'un oligoside selon l'invention comme ingrédient actif dans la préparation d'une composition pharmaceutique destinée à renforcer les défenses immunitaires d'un individu ;

- Un procédé de préparation d'une composition pharmaceutique telle que définie précédemment, caractérisé par le fait que (i) on soumet le polyside de départ à une réaction de dépolymérisation par oxydo-réduction, que (ii), si désiré, soit, on couple l'oligoside obtenu avec un partenaire de conjugaison ou un liposome, soit, on l'incorpore dans des liposomes, des iscoms ou des cyclo-dextrines et que (iii) l'on met le produit obtenu sous une forme pharmaceutique appropriée.

Une composition pharmaceutique préférée comprend au moins un oligoside sous forme de conjugué.

La composition pharmaceutique selon l'invention peut être fabriquée de manière conventionnelle. En particulier on associe un oligoside selon l'invention avec un diluant ou un support acceptable d'un point de vue pharmaceutique, par exemple une solution saline physiologique apyrogène. En outre, une composition selon l'invention peut contenir des ingrédients usuels tel qu'un tampon, un agent conservateur ou stabilisant, un adjuvant et le cas échéant, un excipient de lyophilisation. Un oligoside selon l'invention peut être conservé en présence d'un diluant, d'un support ou d'un ingrédient tel que précédemment évoqué. De manière alternative, un diluant, un support ou un ingrédient peut être ajouté juste avant emploi.

C'est pourquoi l'invention propose de manière alternative un kit contenant :

a) une composition pharmaceutique essentiellement constituée d'au moins un oligoside selon l'invention, à titre d'agent actif ;

b) une composition comprenant au moins un élément sélectionné parmi un diluant ou un support acceptable d'un point de vue pharmaceutique, un composé ayant un pouvoir tampon, un agent conservateur ou stabilisant et un adjuvant ;

c) des instructions pour l'administration concomitante e.g., sous forme de mélange des compositions décrites sous a) et b).

Une composition selon l'invention peut être administrée par n'importe quelle voie conventionnelle, en particulier par voie sous-cutanée, par voie intra-musculaire, par voie intra-veineuse, par exemple sous forme de suspension injectable ou par voie orale. L'administration peut avoir lieu en dose unique ou répétée une ou plusieurs fois après un certain délai d'intervalle. Le dosage approprié varie en fonction de divers paramètres, par exemple, de l'individu traité ou du mode d'administration. On indique toutefois que de bons résultats peuvent être obtenus avec une dose unitaire de 1 à 200 μg d'oligoside sous un volume d'environ 0,5 ml.

L'invention est présentée plus en détail dans les exemples suivants et par référence aux Figures 1 à 4.

La Figure 1 présente les formules des unités répétitives du polyside capsulaire de *S. pneumoniae* type 14 (a), type 1 (b), de *S. typhi* (c), de *S. pneumoniae* type 6B (d), d'*H. influenzae* type B (e), de *S. pneumoniae* type 19F (f), de *N. meningitidis* groupe A (g), de *S. pneumoniae* type 18C (h), type 23F (i) *Klebsiella ozaenae* sérotype K4 *(j), Shigella flexneri* sérotype 1b (k) et *Cryptococcus neoformans* souche 98 (l). On remarque que (a) correspond à un polyoside neutre, que (b) et (c) correspondent à des polyosides possédant des acides uroniques dans la chaîne polyosidique, que (d) à (i) correspondent à des polyosides phosphorylés, que (j) et (k) correspondent à des lipopolysaccharides, respectivement anionque et neutre et que (l) correspond à un polyoside fungique anionique.

Les Figures 2a et 2b représentent les profils d'élution des polyosides non-fragmentés de *S. typhi* (2a) et de *S. pneumoniae* type 1 (2b) sur une colonne de Sépharose 4BCL. Les conditions de chromatographie sont comme suit : on applique sur la colonne de Sépharose préalablement équilibrée avec du NaCl 0,2 M, 2 ml d'une solution de polyoside à 5 mg/ml. L'élution est mise en oeuvre avec du NaCl 0,2 M à une vitesse de 42 ml/h. Le profil d'élution est automatiquement analysé en mesurant la densité optique à 206 nm. En 2a et 2b, le volume mort de la colonne est de 76,56 ml tandis que le volume total est de 201,84 ml.

La Figure 3 représente une gamme-étalon de dextran de 500.000 à 20.000.

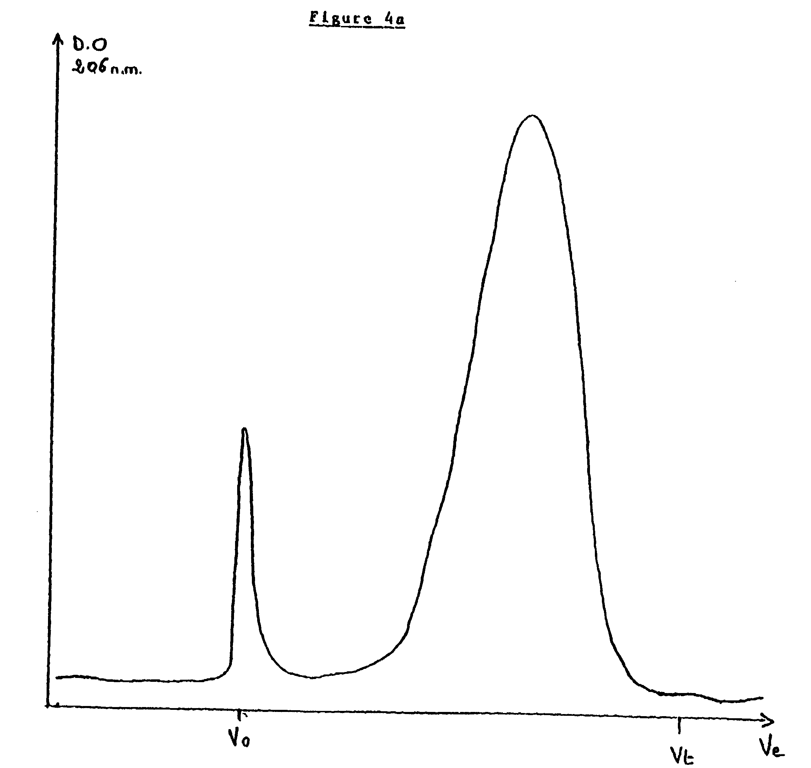

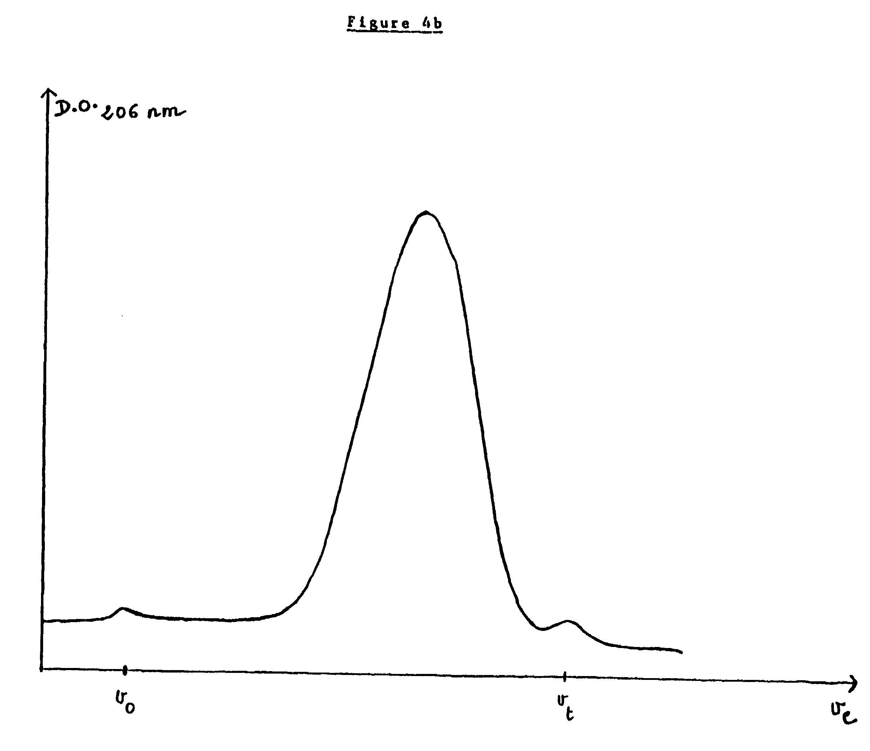

Les Figures 4a et 4b représentent les profils d'élution des oligosides obtenus par fragmentation oxydo-réductive des polyosides de *S. typhi* (4a) et de *S. pneumoniae* type 1 (4b) sur une colonne de Sépharose 4BCL. Les conditions de chromatographie sont telles que précédemment énoncées dans la description de la Figure 2.

Exemple 1 : Fragmentation du polyoside Vi de *S.typhi* en présence d'acide ascorbique.

Une poudre sèche du polyoside Vi obtenue selon la méthode de Gotschlich et al, Prog. Immunobiol. Standard. (1972) 5 : 485 est reprise dans de l'eau apyrogène à raison de 1 mg/ml. Le poids moléculaire (PM) moyen du polyoside correspond à un $K_D$ nul sur une colonne de Sépharose 4BCL .

A 500 ml de cette préparation pré-chauffée à 37°C, on ajoute lentement, en continu pendant 2 h, 12,5 ml d'une solution aqueuse contenant 100 mM d'acide ascorbique, 10 mM de $CuSO_4$ et 10 mM de $FeSO_4$ ; soit une concentration finale d'acide ascorbique de 0,44 mg/ml. Le pH du milieu réactionnel ainsi obtenu est d'environ pH 4. La réaction est poursuivie sous agitation douce environ 3 h (temps d'addition du réactif compris) à 37°C.

Le mélange réactionnel est ensuite filtré sur une membrane d'ultrafiltration 3K (seuil de coupure : 3 kilodaltons). Le rétentat est lavé une première fois avec une solution de NaCl 0,5 M, puis une seconde fois avec de l'eau. Le rétentat est repris dans environ 100 ml d'eau, puis lyophilisé pour conservation.

Le taux de clivage est de l'ordre de 95 % tel que calculé par intégration des courbes obtenues par filtration sur gel de Sépharose 4BCL (Figure 4.a). Par ailleurs, on estime le PM moyen de l'oligoside ainsi obtenu par la méthode de Granath & al (supra). Celui-ci, exprimé en constante d'élution moyenne est de l'ordre de 0,6, soit un PM de 60 000 EqD (en prenant pour référence le dextran).

L'analyse de l'oligoside en spectrométrie RMN (résonance magnétique nucléaire) indique clairement que la structure de l'unité répétitive caractéristique du polyoside a été conservée après fragmentation. En particulier, il n'y a pas eu de perte de groupements fonctionnels ramifiés ou de sucres du squelette polyosidique.

Les fonctions chimiques caractéristiques de l'unité répétitive du polyoside Vi sont analysées par dosage colorimétrique. Le groupement O-acétyle en position ramifiée est dosé par la méthode de Hestrin, Biol. Chem. (1949) 188 : 249 tandis que le polyacide de la structure linéaire est dosé par la méthode de Stone et al, Clin. J. Microbiol. (1988) 28 : 719. Les dosages sont effectués en parallèle sur du polyoside Vi non-fragmenté et sur l'oligoside formé.

Les rapports [O-acétyle] / [polyacide] mesurés pour le polyoside et l'oligoside sont identiques. Ceci montre bien que la méthode de fragmentation du polyoside a permis de conserver la totalité des groupements O-acétyle labiles.

Exemple 2 : Fragmentation du polyoside Vi de *S.typhi* en présence de peroxyde d'hydrogène ($H_2O_2$).

Une poudre sèche du polyoside Vi obtenu selon la méthode de Gotschlich et al, (supra) est reprise en tampon phosphate 0,2 M pH 7,5 à raison de 0,4 mg/ml. Le PM du polyoside correspond à un $K_D$ nul sur une colonne de Sépharose 4BCL.

A 100 ml de cette préparation pré-chauffée à 37°C, on ajoute lentement, sous agitation douce, 11 ml d'une solution aqueuse contenant 0,3 mg/ml d'$H_2O_2$ et 1,1 ml d'une solution de $CuSO_4$ à 2,6 mg/ml. La réaction est poursuivie sous agitation douce environ 1 h à 37° C.

L'expérience est renouvelée dans les même conditions à l'exception du temps de réaction qui est de 2 heures au lieu d'une.

Dans les deux cas, chaque oligoside ainsi obtenu est ensuite recueilli et purifié tel que décrit dans l'Exemple 1.

De même, les caractéristiques des oligosides ainsi obtenus sont mises en évidence selon les méthodes citées dans l'Exemple 1. Les résultats sont comme suit :

- Dans les deux cas, le taux de clivage du polyoside Vi est de l'ordre de 100 %.

- Le PM moyen des oligosides formés après une et deux heures correspond respectivement à un $K_D$ de l'ordre de 0,7 et 0,8 ; soit 30.000 et 10.000 EqD.

- Dans les deux cas, on ne constate pas de modification dans la structure de l'unité répétitive.

Exemple 3 : Fragmentation du polyoside de *N. meningitidis* groupe A

Une poudre sèche du polyoside de *N. meningitidis* groupe A telle que obtenue par la méthode de Gotschlich et al (supra) est reprise en tampon phosphate 0,2 M pH 7,5 soit à raison de 1 mg/ml, soit à raison de 5 mg/ml. Le PM moyen du polyoside correspond à un $K_D$ de 0,15 tel que mesuré sur une colonne de Sépharose 4BCL.

Les préparations à 1 et 5 mg/ml sont pré-chauffées à 37°C.

3a) A 100 ml de la préparation à 1 mg/ml, on ajoute 0,75 ml d'une solution réactionnelle contenant 100 mM d'acide ascorbique, 10 mM de $CuSO_4$ et 10 mM de $FeSO_4$. La réaction est poursuivie sous agitation 1 h 30 à 37°C. Puis on ajoute à nouveau 0,75 ml de la solution réactionnelle ; on obtient ainsi une concentration finale d'acide ascorbique de 0,26 mg/ml. La réaction est poursuivie à nouveau pendant 1 h 30 dans les mêmes conditions.

3b) A 100 ml de la préparation à 5 mg/ml, on ajoute 1,5 ml d'une solution réactionnelle contenant 100 mM d'acide ascorbique, 10 mM de $CuSO_4$ et 10 mM de $FeSO_4$; soit une concentration finale d'acide ascorbique de 0,26 mg/ml. La réaction est poursuivie sous agitation 1 h 30 à 37°C.

Les oligosides obtenus en 3a) et 3b) sont recueillis et purifiés tel que décrit dans l'Exemple 1. De même, les analyses sont mises en oeuvre tel que mentionné dans l'Exemple 1. Dans les deux cas, on observe un taux de clivage de 100 %, sans détérioration de la structure de l'unité répétitive comme analysée par RMN. Le PM moyen de l'oligoside obtenu en 3a) correspond à un $K_D$ de 0,7 (soit 30.000 EqD) tandis que le PM moyen de l'oligoside obtenu en 3b) correspond à un $K_D$ de l'ordre de 0,4 (soit 110.000 EqD).

L'ensemble de ces résultats indique qu'en faisant varier les conditions expérimentales (concentration en polyoside et en agent réactif oxydo-réducteur ainsi que le mode d'addition de l'agent réactif et le temps de réaction) on peut obtenir des oligosides de PM moyen substantiellement différent, tout en conservant un taux de clivage de 100%.

Enfin cet exemple ainsi que l'Exemple 2, montrent bien qu'il s'agit d'une fragmentation par oxydo-réduction et non par hydrolyse acide ou basique dans la mesure où cette fragmentation peut être mise en oeuvre à un pH proche de la neutralité.

Exemple 4 : Fragmentation du polyoside de *S. pneumoniae* des types 1 et 19F et d'*H. influenzae* du type B, en milieu tamponné.

L'Exemple 3 (3a et 3b) est répété en utilisant les polyosides de *S. pneumoniae* types 1 et 19F tels que obtenus selon la méthode décrite dans la demande de brevet française FR 2 495 939 publiée le 18 juin 1982 ainsi que le polyoside d'*H. infuenzae* type B obtenu selon la méthode de Gotschlich et al (supra) ;

Les résultats sont comme suit :

| Souche | PM moyen du polyoside (exprimé en $K_D$) | Protocole a) | | Protocole b) | |
|---|---|---|---|---|---|
| | | PM moyen de l'oligoside | Taux de clivage | PM moyen de l'oligoside | Taux de clivage |
| S.pneumonize 1 | 0,04 | 0,84 | 100 | 0,35 | 100 |
| S.pneumonize 19F | 0,04 | 0,80 | 100 | 0,38 | 100 |
| H.influenze B | 0,1 | 0,75 | 100 | ND | ND |

ND : non determiné

L'analyse de l'oligoside en spectrométrie RMN (résonance magnétique nucléaire) indique clairement que la structure des unités répétitives caractéristiques des polyosides a été conservée après fragmentation.

Exemple 5 : Fragmentation des polyosides *N. meningitidis* groupe A et de *S. pneumoniae* types 1, 14, 18C, 19F et 23F en milieu non-tamponné.

Les préparations de polyosides ainsi que les réactions sont mises en oeuvre comme décrit dans les Exemples 3 et 4 compte tenu des exceptions suivantes : (i) les préparations de polyosides sont préparées à 5 mg/ml en eau apyrogène, (ii) la solution réactionnelle est ajoutée une seule fois à une concentration finale de 2,5 mg/ml et (ii) la réaction est poursuivie seulement pendant 1 heure.

Les oligosides obtenus sont recueillis et purifiés tel que décrit dans l'Exemple 1. Leurs caractéristiques sont évaluées ainsi que mentionné dans l'Exemple 1. Le taux de clivage de chacun des polyosides est de 100 % sans qu'il y ait détérioration de la structure des unités répétitives. Ce dernier point a également été mis en évidence par dosage colorimétrique : Les hexoses sont dosés par la méthode de Scott et al, Anal. Chem. (1953) 25 : 1650, les hexosamines par la méthode de Gatt et al, Anal. Biochem. (1965) 15 : 167, le rhamnose par la méthode de Dische et al, J. Biol. Chem. (1948) 175 : 595 et le phosphate par la méthode de Chen et al, Anal. Chem. (1956) 28 ; 1756. En ce qui concerne le polyoside de *S. pneumoniae* type 14 et l'oligoside qui en est dérivé par fragmentation, les rapports [hexose] /[hexosamine] sont substantiellement identiques. Il en est de même des rapports [rhamnose]/[hexose] et [phosphate] /[hexose] respectivement considérés dans le cas de *S. pneumoniae* types 18C et 23F.

L'analyse par spectrométrie RMN des oligosides ne met pas en évidence de signaux indiquant une hétérogénéité dans les unités répétitives et montre ainsi que les oligosides sont constitués d'une unité répétitive intacte.

Enfin, en ce qui concerne le PM moyen des oligosides, les résultats sont comme suit :

| Souche | PM moyen du polyoside (exprimé en $K_D$) | PM moyen de l'oligoside (exprimé en $K_D$) |
|---|---|---|
| *N.meningitidis* A | 0,15 | 0,66 |
| *S.pneumoniae* 1 | 0,04 | 0,72 |
| *S.pneumoniae* 14 | 0,01 | 0,72 |
| *S.pneumoniae* 18C | 0,03 | 0,75 |
| *S.pneumoniae* 19F | 0,04 | 0,67 |
| *S.pneumoniae* 23F | 0,07 | 0,57 |

Exemple 6 : Préparation d'un conjugué oligoside Vi de *S. typhi* /sous-unité B de la toxine cholérique.

6a) Introduction d'un groupe $NH_2$ sur l'oligoside $V_i$.

Un lyophilisât de l'oligoside Vi tel qu'obtenu dans l'Exemple 1 est repris dans un tampon phosphate pH 8 à raison de 10 mg/ml. A cette préparation, on ajoute du diaminohexane et du NaCNBH, en solutions ; chacun à une concentration finale de 12,5 mg/ml. La réaction est poursuivie pendant 6 jours à température ambiante. La préparation est ensuite dialysée contre de l'eau et lyophilisée.

Par dosage selon la méthode de Shnyder et al, Anal. Biochem. (1975) 64 : 284, on contrôle que des groupes $NH_2$ ont bien été introduits. De même, par dosage selon Hestrin, on montre que les groupes O-acétyles sont toujours conservés.

6b) Introduction d'une fonction réactive.

Le lyophilisât obtenu en 6a) est repris en solution à raison de 40 mg/ml. A 20 ml de cette préparation, on ajoute 80 ml d'une solution à 40 mg/ml de disuccinimidyl subérate (DSS) en diméthylsulfoxyde (DMSO). La réaction est poursuivie pendant une heure à température ambiante. La préparation est ensuite précipitée dans un mélange de DMSO/dioxane.

6c) Conjugaison.

Le précipitat obtenu en 6b) est repris dans une solution de NaCl 0,5 M à raison de 40 mg/ml. En parallèle, on prépare une solution de sous-unité B de la toxine cholérique (Tayot et al, Eur. J. Biochem. (1981) 113 : 249) en tampon phosphate 0,2 M pH 6,5 à raison de 10 mg/ml.

Les deux solutions ainsi préparées sont mélangées ensemble. Le rapport pondéral oligoside/protéine est de 1 environ. La réaction est poursuivie à température ambiante pendant 15 heures.

6d) Purification.

Le mélange est ensuite filtré sur une minicassette d'ultrafiltration (Millipore) équipée d'une membrane dont le seuil de séparation est de 5.10 daltons, afin d'éliminer la protéine et l'oligoside non-couplé. Le rétentat est lavé en NaCl 0,3 M puis conservé à raison de 200 μg/ml en NaCl 8/1.000, merthiolate 1/10.000, à 4°C, après filtration stérile.

Exemple 7 : Préparation de conjugués oligoside/anatoxine tétanique.

On conjugue les oligosides tels qu'obtenus à partir des polyosides de *S. pneumoniae* types 14 et 23F dans l'Exemple 5, ainsi que l'oligoside obtenu à partir du polyoside Vi de *S.typhi* dans l'Exemple 1, en opérant de manière analogue au protocole reporté dans l'Exemple 6 et en remplaçant la sous-unité B de la toxine cholérique par l'anatoxine tétanique préparée selon la méthode de Mueller et Miller, J. Bact. (1954) 67 : 671.

Les conjugués obtenus à partir d'oligosides de *S. pneumoniae* sont conservés à 250 µg/ml.

Exemple 8 : Mise en évidence du pouvoir immunogène des conjugués.

Des souris OF1 (Iffa Credo) sont réparties par lot de 8. Chaque souris d'un lot reçoit 0,5 ml d'une des solutions préparées aux Exemples 6 et 7 ou en parallèle, 0,5ml des polyosides natifs correspondants. Les injections s'effectuent par voie sous-cutanée. Ces injections sont répétées 14 jours après. En parallèle, un lot témoin reçoit uniquement de l'eau physiologique.

14 et 28 jours après la première injection, on effectue un prélèvement sanguin et on titre les anticorps IgG anti-polyoside par dosage Elisa (les plaques de titrage sont recouvertes de polyoside non-fragmenté). Le taux d'anticorps à 14 et 28 jours montre que les conjugués sont immunogènes. Dans chacun des cas, le taux d'anticorps est supérieur à celui induit par le polyoside natif correspondant. De plus on observe un effet rebond caractéristique d'un antigène T-dépendant.

Exemple 9 : Composition pharmaceutique vaccinale comprenant des conjugués à titre d'agents actifs.

Les conjugués oligosides Vi de S.typhi/anatoxine tétanique obtenus selon l'Exemple 7 sont formulés dans une dose vaccinale de 0,5 ml destinée à être injectée chez l'homme par voie sous-cutanée. La composition par dose étant :

- Oligosides Vi de S.typhi conjugués     10 µg
- Tampon phosphate à pH 7       475 µg (en ions$PO_4$)
- Nacl     4250 µg
- Merthiolate       0,05 µg
- Eau p.p.i.       qsp 0,5 ml

**Revendications**

1. Procédé de préparation d'un oligoside par dépolymérisation d'un polyoside antigénique, issu d'un agent pathogène, dont les unités répétitives contiennent au moins une fonction chimique labile, en conservant la structure des unités répétitives avec leurs fonctions labiles, lesdites fonctions chimiques labiles étant définies comme des fonctions qui, lorsque le polyoside est soumis à un procédé de dépolymérisation de polyosides par fragmentation aux ultrasons ou par hydrolyse acide ou basique, subissent une modification altérant l'antigénicité, dans lequel :

    (i) on soumet ledit polyoside à une réaction de dépolymérisation par oxydo-réduction, et
    (ii) on récolte l'oligoside ainsi obtenu.

2. Procédé selon la revendication 1, **caractérisé en ce que** l'on effectue la réaction de dépolymérisation par oxydo-réduction en présence d'un agent oxydo-réducteur qui est l'acide ascorbique ou le peroxyde d'hydrogène.

3. Procédé selon la revendication 2, **caractérisé en ce que** l'on effectue la réaction de dépolymérisation par oxydo-réduction en présence d'un sel de fer ou de cuivre.

4. Procédé selon l'une quelconque des revendications précédentes, **caractérisé en ce que** l'on met en oeuvre la réaction de dépolymérisation par oxydo-réduction sur une solution aqueuse contenant de 0,5 à 50 mg/mL dudit polyoside.

5. Procédé selon la revendication précédente, dans lequel ladite solution contient de 0,5 à 10 mg/mL dudit polyoside.

6. Procédé selon la revendication 4 ou 5, **caractérisé en ce que** ladite solution a un pH de 3 à 8,5.

7. Procédé selon la revendication précédente, dans lequel ladite solution a un pH de 3 à 6,5.

8. Procédé selon l'une quelconque des revendications précédentes, **caractérisé en ce que** l'on met en oeuvre la réaction de dépolymérisation par oxydo-réduction à une température pouvant varier de 4 à 60°C.

9. Procédé selon la revendication précédente, dans lequel ladite température peut varier de 18 à 40°C.

10. Procédé selon l'une quelconque des revendications précédentes, **caractérisé en ce que** l'agent oxydo-réducteur est l'acide ascorbique utilisé à une concentration de 0,01 à 25 mg/mL.

11. Procédé selon la revendication précédente, dans lequel l'acide ascorbique est utilisé à une concentration de 0,1 à 10 mg/mL.

12. Procédé selon la revendication 3 ou selon la revendication 3 et l'une quelconque des revendications 4 à 11, **caractérisé en ce que** lesdits sels métalliques sont présents à une concentration de 1 mM à 100 .mM.

13. Procédé selon la revendication précédente, dans lequel lesdits sels métalliques sont présents à une concentration de 1 à 10 mM.

14. Procédé selon l'une quelconque des revendications 1 à 11, **caractérisé en ce que** l'agent oxydo-réducteur est le peroxyde d'hydrogène, utilisé à une concentration de 0,1 à 100 mM.

15. Procédé selon la revendication précédente, dans lequel le peroxyde d'hydrogène est utilisé à une concentration de 0,5 à 10 mM.

16. Procédé selon l'une quelconque des revendications précédentes, **caractérisé par le fait que** ladite fonction chimique labile est choisie parmi les groupements phosphate, acétyle et pyruvate,

17. Procédé selon l'une quelconque des revendications 1 à 16, **caractérisé en ce que** ledit polyoside antigénique est un polyoside capsulaire issu d'une bactérie pathogène.

18. Procédé selon la revendication 17, **caractérisé en ce que** ladite bactérie pathogène est sélectionnée parmi celles des genres *Staphylococcus, Streptococcus, Klebsiella, Salmonella, Escherichia, Neisseria* et *Haemophilus.*

19. Procédé selon la revendication 18, **caractérisé en ce que** ladite bactérie pathogène est sélectionnée parmi *S. typhi, S. pneumoniae, N, meningitidis* et *Haemophilus influenzae.*

20. Procédé selon l'une des revendications 1 à 19, **caractérisé en ce que** l'on récolte un oligoside obtenu ayant une constante d'élution moyenne, telle que mesurée sur une colonne Sépharose 4BCL, de 0,2 à 1.

21. Procédé selon la revendication 20, **caractérisé en ce que** l'on récolte un oligoside obtenu ayant une constante d'élution moyenne, telle que mesurée sur une colonne Sépharose 4BCL, de 0,4 à 0,8.

22. Procédé selon l'une des revendications 1 à 21, **caractérisé en ce qu'**il comprend en outre l'étape consistant (iii) soit à coupler l'oligoside obtenu en (ii) à un partenaire de conjugaison ou à un porteur pour obtenir ledit oligoside sous la forme d'un conjugué, ou lié de manière covalente à un porteur, soit à incorporer l'oligoside obtenu en (ii) dans un vecteur.

23. Procédé selon la revendication 22, **caractérisé en ce que** ledit partenaire de conjugaison est une protéine bactérienne.

24. Procédé selon la revendication 23, **caractérisé en ce que** ladite protéine bactérienne est une toxine, une anatoxine, une sous-unité d'une toxine multimérique, une protéine de membrane ou une sous-unité d'une protéine de membrane multimérique.

25. Procédé selon la revendication 22, **caractérisé en ce que** le porteur est un liposome.

26. Procédé selon la revendication 22, **caractérisé en ce que** le vecteur est choisi parmi les liposomes et les ISCOMS.

27. Composition pharmaceutique comprenant à titre d'agent actif au moins un oligoside obtenu par le procédé selon l'une des revendications 1 à 26.

28. Composition selon la revendication 27, **caractérisée en ce qu'**elle constitue une composition vaccinale.

29. Utilisation d'un oligoside obtenu par le procédé selon l'une des revendications 1 à 26, comme ingrédient actif dans la préparation d'une composition pharmaceutique destinée à renforcer les défenses immunitaires d'un individu, et en particulier à réaliser une vaccination.

**Claims**

1. Process for preparing an oligosaccharide by depolymerization of an antigenic polysaccharide obtained from a pathogenic agent, whose repeating units contain at least one labile chemical functional group, while preserving the structure of the repeating units with their labile functional groups, the said labile chemical functional groups being defined as functional groups which, when the polysaccharides is subjected to a process for the depolymerization of polysaccharide by fragmentation with ultrasound or by acid or basic hydrolysis, undergo modification which impairs antigenicity, in which:

   (i) the said polysaccharide is subjected to an oxidation-reduction depolymerization reaction, and
   (ii) the oligosaccharide thus obtained is recovered.

2. Process according to Claim 1, **characterized in that** the oxidation-reduction depolymerization reaction is carried out in the presence of an oxidation-reduction agent which is ascorbic acid or hydrogen peroxide.

3. Process according to Claim 2, **characterized in that** the oxidation-reduction depolymerization reaction is carried out in the presence of an iron or copper salt.

4. Process according to any one of the preceding claims, **characterized in that** the oxidation-reduction depolymerization reaction is carried out on an aqueous solution containing 0.5 to 50 mg/mL of the said polysaccharide.

5. Process according to the preceding claim, in which the said solution contains from 0.5 to 10 mg/mL of the said polysaccharide.

6. Process according to Claim 4 or 5, **characterized in that** the said solution has a pH of 3 to 8.5.

7. Process according to the preceding claim, in which the said solution has a pH of 3 to 6.5.

8. Process according to any one of the preceding claims, **characterized in that** the oxidation-reduction depolymerization reaction is carried out at a temperature which may vary from 4 to 60°C.

9. Process according to the preceding claim, in which the said temperature may vary from 18 to 40°C.

10. Process according to any one of the preceding claims, **characterized in that** the oxidation-reduction agent is ascorbic acid used at a concentration of 0.01 to 25 mg/mL.

11. Process according to the preceding claim, in which the ascorbic acid is used at a concentration of 0.1 to 10 mg/mL.

12. Process according to Claim 3 or according to Claim 3 and any one of Claims 4 to 11, **characterized in that** the said metal salts are present at a concentration of 1 mM to 100 mM.

13. Process according to the preceding claim, in which the said metal salts are present at a concentration of 1 to 10 mM.

14. Process according to any one of Claims 1 to 9, **characterized in that** the oxidation-reduction agent is hydrogen peroxide, used at a concentration of 0.1 to 100 mM.

15. Process according to the preceding claim, in which the hydrogen peroxide is used at a concentration of 0.5 to 10 mM.

16. Process according to any one of the preceding claims, **characterized in that** the said labile chemical functional group is chosen from phosphate, acetyl and pyruvate groups.

17. Process according to any one of Claims 1 to 16, **characterized in that** the said antigenic polysaccharide is a capsular polysaccharide obtained from a pathogenic bacterium.

18. Process according to Claim 17, **characterized in that** the said pathogenic bacterium is selected from those of the genera *Staphylococcus, Streptococcus, Klebsiella, Salmonella, Escherichia, Neisseria* and *Haemophilus.*

19. Process according to Claim 18, **characterized in that** the said pathogenic bacterium is selected from *S. typhi, S. pneumoniae, N, meningitidis* and *Haemophilus influenzae.*

20. Process according to one of Claims 1 to 19, **characterized in that** an oligosaccharide obtained having a mean elution constant, as measured on a Sepharose 4BCL column, of 0.2 to 1 is recovered.

21. Process according to Claim 20, **characterized in that** an oligosaccharide obtained having a mean elution constant, as measured on a Sepharose 4BCL column, of 0.4 to 8 is recovered.

22. Process according to one of Claims 1 to 21, **characterized in that** it comprises, in addition, a step consisting (iii) either in coupling the oligosaccharide obtained in (ii) with a conjugation partner or with a carrier in order to obtain the said oligosaccharide in the form of a conjugate, or covalently bound to a carrier, or in incorporating the oligosaccharide obtained in (ii) into a vector.

23. Process according to Claim 22, **characterized in that** the said conjugation partner is a bacterial protein.

24. Process according to Claim 23, **characterized in that** the said bacterial protein is a toxin, a toxoid, a subunit of a multimeric toxin, a membrane protein or a subunit of a multimeric membrane protein.

25. Process according to Claim 22, **characterized in that** the carrier is a liposome.

26. Process according to Claim 22, **characterized in that** the vector is chosen from liposomes and ISCOMS.

27. Pharmaceutical composition comprising, as active agent, at least one oligosaccharide obtained by the process according to one of Claims 1 to 26.

28. Composition according to Claim 27, **characterized in that** it constitutes a vaccinal composition.

29. Use of an oligosaccharide obtained by the process according to one of Claims 1 to 26, as active ingredient in the preparation of a pharmaceutical composition intended to reinforce the immune defences of an individual, and in particular to carry out a vaccination.

**Patentansprüche**

1. Verfahren zur Herstellung eines Oligosids durch Depolymerisation eines antigenischen Polyosids, das von einem Pathogen stammt, wobei die wiederkehrenden Einheiten mindestens eine chemisch labile Funktion enthalten, bei dem man die Struktur der wiederkehrenden Einheiten mit ihren labilen Funktionen erhält, wobei die chemisch labilen Funktionen als Funktionen definiert sind, die dann, wenn das Polyosid einem Verfahren der Depolymerisation des Polyosids durch Fragmentation mit Ultraschall oder über saure oder basische Hydrolyse unterworfen wird, diese eine die Antigenizität ändernde Modifikation durchlaufen, in dem man:

(i) man das Polyosid einer Depolymerisationsreaktion durch Oxidoreduktion unterwirft und
(ii) man das so erhaltene Oligosid sammelt.

2. Verfahren gemäß Anspruch 1, **dadurch gekennzeichnet, dass** man die Depolymerisationsreaktion durch Oxidoreduktion in Anwesenheit eines Oxidations-/Reduktionsmittels bewirkt, bei dem es sich um Ascorbinsäure oder Wasserstoffperoxid handelt.

3. Verfahren gemäß Anspruch 2, **dadurch gekennzeichnet, dass** man die Depolymerisationsreaktion durch Oxidoreduktion in Anwesenheit eines Eisen- oder Kupfersalzes bewirkt.

4. Verfahren gemäß einem der vorstehenden Ansprüche, **dadurch gekennzeichnet, dass** man die Depolymerisationsreaktion durch Oxidoreduktion an einer wässrigen Lösung, enthaltend 0,5 bis 50 mg/ml des Polyosids, ausführt.

5. Verfahren gemäß dem vorstehenden Anspruch, worin die besagte Lösung 0,5 bis 10 mg/ml des Polyosids enthält.

6. Verfahren gemäß Anspruch 4 oder 5, **dadurch gekennzeichnet, dass** die Lösung einen pH-Wert von 3 bis 8,5 aufweist.

7. Verfahren gemäß dem vorstehenden Anspruch, worin die besagte Lösung einen pH-Wert von 3 bis 6,5 aufweist.

8. Verfahren gemäß einem der vorstehenden Ansprüche, **dadurch gekennzeichnet, dass** man die Depolymerisationsreaktion durch Oxidoreduktion bei einer Temperatur ausführt, die zwischen 4 und 60 °C schwanken kann.

9. Verfahren gemäß dem vorstehenden Anspruch, worin die besagte Temperatur von 18 bis 40 °C schwanken kann.

10. Verfahren gemäß einem der vorstehenden Ansprüche, **dadurch gekennzeichnet, dass** es sich bei dem Oxidations-/Reduktionsmittel um Ascorbinsäure handelt, die in einer Konzentration von 0,01 bis 25 mg/ml verwendet wird.

11. Verfahren gemäß dem vorstehenden Anspruch, worin die Ascorbinsäure in einer Konzentration von 0,1 bis 10 mg/ml verwendet wird.

12. Verfahren gemäß Anspruch 3 oder gemäß Anspruch 3 und einem der Ansprüche 4 bis 11, **dadurch gekennzeichnet, dass** die Metallsalze in einer Konzentration von 1 mM bis 100 mM vorhanden sind.

13. Verfahren gemäß dem vorstehenden Anspruch, worin die besagten Metallsalze in einer Konzentration von 1 bis 10 mM vorhanden sind.

14. Verfahren gemäß einem der Ansprüche 1 bis 11 (9), **dadurch gekennzeichnet, dass** das Oxidations-/Reduktionsmittel Wasserstoffperoxid ist, das in einer Konzentration von 0,1 bis 100 mM verwendet wird.

15. Verfahren gemäß dem vorstehenden Anspruch, worin das Wasserstoffperoxid in einer Konzentration von 0,5 bis 10 mM verwendet wird.

16. Verfahren gemäß einem der vorstehenden Ansprüche, **dadurch gekennzeichnet, dass** die chemisch labile Funktion ausgewählt ist aus den Phosphat-, Acetyl- und Pyruvatgruppen.

17. Verfahren gemäß einem der Ansprüche 1 bis 16, **dadurch gekennzeichnet, dass** das antigenische Polyosid ein Hüll-Polyosid ist, das von einem pathogenen Bakterium stammt.

18. Verfahren gemäß Anspruch 17, **dadurch gekennzeichnet, dass** das pathogene Bakterium ausgewählt ist aus denjenigen der Gattungen *Staphylococcus, Streptococcus, Klebsiella, Salmonella, Escherichia, Neisseria* und *Haemophilus.*

19. Verfahren gemäß Anspruch 18, **dadurch gekennzeichnet, dass** das pathogene Bakterium ausgewählt ist unter *S. typhi, S. pneumoniae, N. meningitidis* und *Haemophilus influenzae.*

20. Verfahren gemäß einem der Ansprüche 1 bis 19, **dadurch gekennzeichnet, dass** man ein Oligosid sammelt, erhalten mit einer mittleren Elutionskonstante von 0,2 bis 1, wie sie auf einer Sepharose-4BCL-Säule gemessen wird.

21. Verfahren gemäß Anspruch 20, **dadurch gekennzeichnet, dass** dass man ein Oligosid sammelt, erhalten mit einer mittleren Elutionskonstante von 0,4 bis 0,8, wie sie auf einer Sepharose-4BCL-Säule gemessen wird.

**22.** Verfahren gemäß einem der Ansprüche 1 bis 21, **dadurch gekennzeichnet, dass** es zusätzlich den Schritt enthält, bestehend aus (iii) entweder dem Ankuppeln des im Schritt (ii) erhaltenen Oligosids an einen Konjugationspartner oder einen Träger zum Erhalt des Oligosids in Form eines Konjugats oder auf kovalente Weise gebunden an einen Träger, oder des Inkorporierens des in Schritt (ii) erhaltenen Oligosids in einen Vektor.

**23.** Verfahren gemäß Anspruch 22, **dadurch gekennzeichnet, dass** es sich bei dem Konjugationspartner um ein bakterielles Protein handelt.

**24.** Verfahren gemäß Anspruch 23, **dadurch gekennzeichnet, dass** es sich bei dem bakteriellen Protein um ein Toxin, ein Anatoxin, eine Untereinheit eines multimeren Toxins, ein Membranprotein oder eine Untereinheit eines multimeren Membranproteins handelt.

**25.** Verfahren gemäß Anspruch 22, **dadurch gekennzeichnet, dass** es sich bei dem Träger um ein Liposom handelt.

**26.** Verfahren gemäß Anspruch 22, **dadurch gekennzeichnet, dass** der Vektor ausgewählt ist unter den Liposomen und den ISCOMS.

**27.** Pharmazeutische Zubereitung, enthaltend als aktives Mittel mindestens ein Oligosid, erhalten über das Verfahren gemäß einem der Ansprüche 1 bis 26.

**28.** Zubereitung gemäß Anspruch 27, **dadurch gekennzeichnet, dass** sie eine Vakzin-Zubereitung darstellt.

**29.** Verwendung eines Oligosids, erhalten über ein Verfahren gemäß einem der Ansprüche 1 bis 26, als aktiver Bestandteil bei der Herstellung einer pharmazeutischen Zubereitung, bestimmt zum Verstärken der Immunantworten in einem Individuum und insbesondere zur Realisierung einer Impfung.

## FIGURE 1

a) $\left[\text{->4)-}\beta\text{-D-Glc}p\text{-(1->6)-}\beta\text{-D-Glc}p\text{NAc-(1->3)-}\beta\text{-D-Gal}p\text{-(1}\underset{\underset{\beta\text{-D-Gal}p}{|}}{\overset{4}{\phantom{x}}}\right]_n$

b) $\left[\text{->3)-}\alpha\text{-Sug}p\text{-(1->4)-}\alpha\text{-D-Gal}p\text{A-(1->3)-}\alpha\text{-D-Gal}p\text{A-(1}\right]_n$

c) $\left[\text{->4)-}\alpha\text{D-GalNacA}\underset{\underset{\text{OAc}}{|}}{\overset{3|}{\phantom{x}}}\right]_n$

d) $\left[\text{->2)-}\alpha\text{-D-Gal-(1->3)-}\alpha\text{-D-Glc-(1->3)-}\alpha\text{-L-Rha-(1->4)-D-ribitol-(5-0-P-0}\overset{0}{\underset{\text{OH}}{|}}\right]_n$

e) $\left[\text{->3)-}\beta\text{-D-Ribf-(1->1)-D-ribitol-(5-0-P-0}\overset{O}{\underset{\text{OH}}{|}}\right]_n$

f) $\left[\text{>4-}\beta\text{-D-Man}p\text{Nac-(1->4)-}\alpha\text{-D-Glc}p\text{-(1->2)-}\alpha\text{-L-Rha}p\text{-(1-0-P-0}\overset{O}{\underset{\text{OH}}{\|}}\right]_n$

g) $\left[\text{->6)-(}\alpha\text{-D-Man}p\text{Nac-(1-0-P-0}\underset{\underset{\text{OAc}}{|}}{\overset{3|}{\phantom{x}}}\overset{0}{\underset{\text{OH}}{\|}}\right]_n$

h) $\left[\begin{array}{c}\alpha\text{-D-Glc}p\\1\\|\\2\\\text{->4)}\beta\text{-D-Glc}p\text{(1->4)}\beta\text{-D-Gal}p\text{(1->4)}\alpha\text{-D-Glc}p\text{(1->3)}\alpha\text{-L-Rha}p\text{(1}\\3\\|\\0\\\text{Glycérol-1-P-OH}\\\|\\0\end{array}\right]_n$

Figure 1 (continuation)

i)

$$\left[\begin{array}{c} \alpha\text{-L-Rha}p \\ | \\ | \\ 2 \\ \text{—4)-}\beta\text{-D-Glc}p\text{-(1}\rightarrow\text{4)-}\beta\text{-D-Gal}p\text{-(1}\rightarrow\text{4)-}\beta\text{-L-Rha}p\text{-(1—} \\ 3 \\ | \\ \text{HO—P=O} \\ | \\ \text{O} \\ | \\ \text{HOCH}_2.\text{CH}.\text{CH}_2\text{OH} \end{array}\right]_n$$

j)

$$\begin{array}{c} \alpha\text{-Kd}o \\ 2 \\ \downarrow \\ 4 \\ \text{—3)-}\beta\text{-D-Glc}p\text{-(1}\rightarrow\text{2)-}\alpha\text{-D-Glc}p\text{A-(1}\rightarrow\text{3)-}\alpha\text{-D-Man}p\text{-(1}\rightarrow\text{3)-}\alpha\text{-D-Glc}p\text{-(1—} \\ 3 \\ | \\ \text{OAc} \end{array}$$

k)

$$\begin{array}{c} \text{Glc} \quad\quad\quad\quad\quad\quad\quad 2\text{-O-Ac} \\ | 1 \quad\quad\quad\quad\quad\quad\quad\quad | \\ \downarrow 4 \quad\quad\quad\quad\quad\quad\quad\quad | \\ \text{—3 GlcNAc 1}\rightarrow\text{2 Rha 1}\rightarrow\text{2 Rha 1}\rightarrow\text{3 Rha 1—} \end{array}$$

l)

$$\begin{array}{ccc} \text{Xyl}p & \text{Glc}p\text{A} & \text{Xyl}p \\ 1 & 1 & 1 \\ \downarrow & \downarrow & \downarrow \\ 2 & 2 & 2 \end{array}$$

Man$p$(1→3)-α-D-Man$p$(1→3)-α-D-Man$p$(1-3)-α-D-Man$p$(1-3)-α-D-

$$\begin{array}{c} \text{Glc}p\text{A} \\ 2 \\ \downarrow \end{array}$$

Man$p$(1-3)-α-D-Man$p$(1-3)-α-D-

Figure 2a

Figure 2b

FIGURE 3

Figure 4a

EP 0 562 107 B1

Figure 4b